# EUROPÄISCHE PATENTANMELDUNG

(11) **EP 2 434 779 A2**
(43) Veröffentlichungstag der Anmeldung: **28.03.2012**
(21) Anmeldenummer: 11181751.6
(22) Anmeldetag: 19.09.2011
(51) Int. Cl.: H04R 25/00

(54) **Verfahren zum Anpassen eines Hörgeräts mit Insitu-Audiometrie und Hörgerät**

(30) Priorität: 24.09.2010 DE 102010041337
(71) Anmelder: Siemens Medical Instruments Pte. Ltd., Singapore 139959 (SG)
(72) Erfinder: Müller-Wehlau, Matthias, 91058 Erlangen (DE); Junius, Dirk, 91096 Möhrendorf (DE); Lauer, Johannes, 91083 Baiersdorf (DE)
(74) Vertreter: Maier, Daniel Oliver

(57) **Zusammenfassung**

Die Anpassung eines Hörgeräts soll verbessert und komfortabeler gestaltet werden. Dazu wird ein Verfahren vorgeschlagen, bei dem das Hörgerät individuell für den Nutzer eingestellt (10) und zumindest teilweise in den Gehörgang des Nutzers eingesetzt wird (11). Anschließend wird eine Insitu-Messung (12, 13) der akustischen Impedanz des Hörsystems des Nutzers einschließlich zumindest eines Teils des Gehörgangs des Nutzers mit einer tympanometrischen Methode durchgeführt. Anhand der Ergebnisse der Insitu-Messung (12, 13) kann eine automatische Korrektur (15) der individuellen Einstellung des Hörgeräts erfolgen.

## Beschreibung

Die vorliegende Erfindung betrifft ein Verfahren zum Anpassen eines Hörgeräts an einen Nutzer durch Einstellen des Hörgeräts individuell für den Nutzer und Einsetzen des Hörgeräts zumindest teilweise in den Gehörgang des Nutzers. Darüber hinaus betrifft die vorliegende Erfindung ein Hörgerät mit einer Signalverarbeitungseinrichtung, die individuell für einen Nutzer einstellbar ist, und einem Abschnitt, der in einen Gehörgang des Nutzers einsetzbar ist.

Hörgeräte sind tragbare Hörvorrichtungen, die zur Versorgung von Schwerhörenden dienen. Um den zahlreichen individuellen Bedürfnissen entgegenzukommen, werden unterschiedliche Bauformen von Hörgeräten wie Hinter-dem-Ohr-Hörgeräte (HdO), Hörgerät mit externem Hörer (RIC: receiver in the canal) und In-dem-Ohr-Hörgeräte (IdO), z.B. auch Concha-Hörgeräte oder Kanal-Hörgeräte (ITE, CIC), bereitgestellt. Die beispielhaft aufgeführten Hörgeräte werden am Außenohr oder im Gehörgang getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.

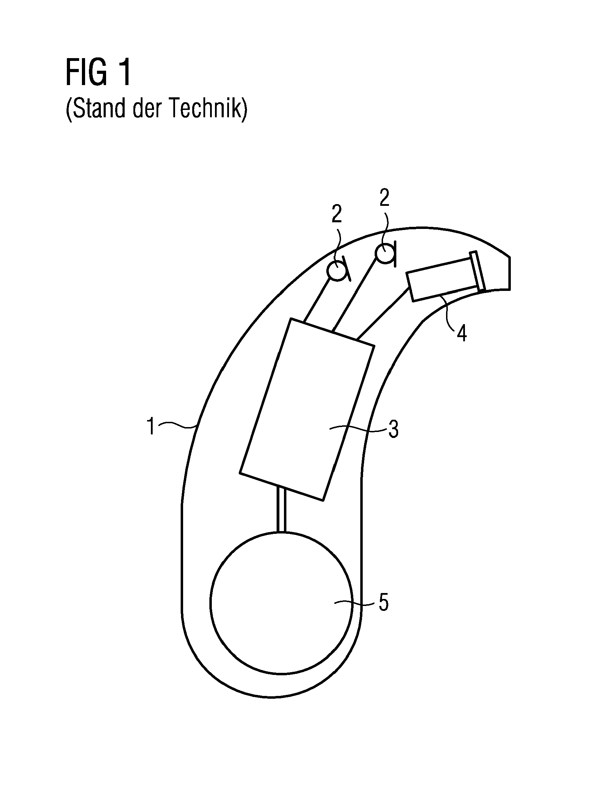

Hörgeräte besitzen prinzipiell als wesentliche Komponenten einen Eingangswandler, einen Verstärker und einen Ausgangswandler. Der Eingangswandler ist in der Regel ein Schallempfänger, z. B. ein Mikrofon, und/oder ein elektromagnetischer Empfänger, z. B. eine Induktionsspule. Der Ausgangswandler ist meist als elektroakustischer Wandler, z. B. Miniaturlautsprecher, oder als elektromechanischer Wandler, z. B. Knochenleitungshörer, realisiert. Der Verstärker ist üblicherweise in eine Signalverarbeitungseinheit integriert. Dieser prinzipielle Aufbau ist in FIG 1 am Beispiel eines Hinter-dem-Ohr-Hörgeräts dargestellt. In ein Hörgerätegehäuse 1 zum Tragen hinter dem Ohr sind ein oder mehrere Mikrofone 2 zur Aufnahme des Schalls aus der Umgebung eingebaut. Eine Signalverarbeitungseinheit 3, die ebenfalls in das Hörgerätegehäuse 1 integriert ist, verarbeitet die Mikrofonsignale und verstärkt sie. Das Ausgangssignal der Signalverarbeitungseinheit 3 wird an einen Lautsprecher bzw. Hörer 4 übertragen, der ein akustisches Signal ausgibt. Der Schall wird gegebenenfalls über einen Schallschlauch, der mit einer Otoplastik im Gehörgang fixiert ist, zum Trommelfell des Geräteträgers übertragen. Die Energieversorgung des Hörgeräts und insbesondere die der Signalverarbeitungseinheit 3 erfolgt durch eine ebenfalls ins Hörgerätegehäuse 1 integrierte Batterie 5.

Hörgeräte werden in erster Linie unter Berücksichtigung des individuellen Audiogramms an den Nutzer angepasst. Moderne Hörgeräte bieten dabei zum Teil die Möglichkeit, das Audiogramm ohne spezielle Geräte nur mit Hilfe des Hörgeräts selber zu ermitteln. Bei dieser Insitu-Audiometrie wird der Test-Ton über den Receiver des Hörgeräts abgegeben und solange im Pegel erhöht, bis der Ton vom Träger des Hörgeräts wahrgenommen wird. Der resultierende Pegel und damit die geschätzte Hörschwelle wird dabei entweder über spezielle Mikrofone im Gehörgang gemessen oder durch Kenntnis des Ausgabepegels des Receivers bei einem kalibrierten Hörgerät geschätzt.

Die geschilderten Methoden zur Einstellung bzw. Anpassung eines Hörgeräts haben zum einen den Nachteil, dass es im Gegensatz zur Ermittlung des Audiogramms mit Hilfe eines Audiometers nicht möglich ist, die Schallleitungs-Hörschwelle zu ermitteln. Die Schallleitungs-Hörschwelle ist ein Maß dafür, inwieweit die akustische Leitung (Luftleitung und Knochenleitung) durch Außenohr und Mittelohr gewährleistet ist. Eine mögliche Schalleitungs-Schwerhörigkeit wird bei der Versorgung mit Hörgeräten grundsätzlich anders behandelt als eine reine sensori-neurale Schwerhörigkeit, bei der das Innenohr geschädigt ist.

Die bekannten Anpassmethoden haben aber auch den Nachteil, dass die Messung des Präsentationspegels durch das Audiogramm bei der Bestimmung der Hörschwelle in der Regel in der Ebene des Hörgeräts und nicht im Bereich des Trommelfells erfolgt. Die sich daraus ergebende frequenzabhängige Abweichung kann unter Umständen die gemessene Hörschwelle verfälschen.

Die Aufgabe der vorliegenden Erfindung besteht somit darin, das Anpassen eines Hörgeräts insbesondere für den Hörgeräteträger zu erleichtern.

Erfindungsgemäß wird diese Aufgabe gelöst durch ein Verfahren zum Anpassen eines Hörgeräts an einen Nutzer durch Einstellen des Hörgeräts individuell für den Nutzer, und Einsetzen des Hörgeräts zumindest teilweise in den Gehörgang des Nutzers, sowie Insitu-Messen der akustischen Impedanz des Hörsystems des Nutzers einschließlich zumindest eines Teils des Gehöhrgangs des Nutzers mit einer tympanometrischen Methode und automatisches Korrigieren der individuellen Einstellung des Hörgeräts anhand des Ergebnisses der Insitu-Messung.

Darüber hinaus wird erfindungsgemäß bereitgestellt ein Hörgerät mit einer Signalverarbeitungseinrichtung, die individuell für einen Nutzer einstellbar ist, und einem Abschnitt, der in einen Gehörgang des Nutzers einsetzbar ist,
sowie mit einer Messeinrichtung, mit der die akustische Impedanz des Hörsystems des Nutzers einschließlich zumindest eines Teils des Gehörgangs des Nutzers im in den Gehörgang eingesetzten Zustand des Abschnitts mit einer tympanometrischen Methode messbar ist, wobei die individuelle Einstellung der Signalverarbeitungseinrichtung anhand der tympanometrischen Messung automatisch korrigierbar ist.

In vorteilhafter Weise ist es so möglich, eine bessere Versorgung unmittelbar am Trommelfell zu erreichen. Dies wird dadurch gewährleistet, dass beim Anpassen die Schallleitung durch Insitu-Messung berücksichtigt wird. Dadurch kann die Einstellung des Hörgeräts sehr individuell an die Gehörgangsform, den Sitz des Hörgeräts im Gehörgang und die Impedanz des Ohrs angepasst werden.

Vorzugsweise erfolgt die individuelle Einstellung des Hörgeräts mit einem Audiogramm. Damit kann eine gute Grundeinstellung des Hörgeräts vor der Benutzung des Hörgeräts erreicht werden.

Das Hörgerät kann einen Vent aufweisen, der für die Insitu-Messung temporär verschlossen wird. Damit kann das Verfahren auch bei offener Versorgung eingesetzt werden.

Besonders vorteilhaft ist, wenn bei der Insitu-Messung ein Test-Ton konstanten Pegels mit einer Frequenz zwischen 200 Hz und 300 Hz von dem Hörgerät abgegeben wird. Vorzugsweise liegt die Frequenz im Bereich zwischen 220 Hz und 230 Hz. Damit ist sie niedrig genug, um im Wesentlichen eine Abhängigkeit von der Steifigkeit der Gehörkomponenten zu erhalten, und hoch genug, um nicht von tieffrequentem Störschall negativ beeinflusst zu werden.

Das Trommelfell kann für die Insitu-Messung vorübergehend schallhart gemacht werden. Dadurch ergibt sich eine veränderte Impedanz, die in erster Linie Informationen über den Gehörgang vor dem Trommelfell liefert.

Aus der Insitu-Messung kann ermittelt werden, ob der Gehörgang in einem vorgegebenen Maß verschlossen ist. Aus dem Tympanogramm kann nämlich sofort erkannt werden, ob das Hörgerät im Gehörgang exakt sitzt oder ob ein ggf. vorhandener Vent für die Messung ausreichend verschlossen ist.

Vorteilhaft ist ferner, wenn eine Differenz von zwei Insitu-Messungen, eine bei schallhartem Trommelfell und die andere
bei nicht schallhartem Trommelfell, zu dem automatischen Korrigieren der Einstellung verwendet wird. Aus dieser Differenz lässt sich nämlich auf den Schallanteil schließen, der in das Mittelohr eindringt. Dies ergibt Informationen über die Funktionsfähigkeit des Mittelohrs. Die Verstärkung des Hörgeräts kann entsprechend frequenzabhängig korrigiert werden.

Das erfindungsgemäße Hörgerät kann in einer Ausführungsform so ausgebildet sein, dass die Messeinrichtung ein Mikrofon umfasst, das in den in den Gehörgang einsetzbaren Abschnitt des Hörgeräts integriert ist. So kann beispielsweise bei einem IdO-Hörgerät ein Mikrofon an der dem Trommelfell zugewandten Seite des Hörgeräts angeordnet sein. Damit sind Insitu-Messungen problemlos möglich.

Die vorliegende Erfindung ist anhand der beigefügten Zeichnungen näher erläutert, in denen zeigen:
- FIG 1: eine schematische Darstellung des Aufbaus eines Hörgeräts gemäß dem Stand der Technik;
- FIG 2: ein Blockschaltdiagramm zu dem erfindungsgemäßen Anpassverfahren und
- FIG 3: ein Tympanogramm

Die nachfolgend geschilderten Ausführungsbeispiele stellen bevorzugte Ausführungsformen der vorliegenden Erfindung dar.

Die der Erfindung zugrunde liegende Idee sieht vor, mit Methoden der Tympanometrie (Messmethode für die Beweglichkeit von Trommelfell und Mittelohr) das Gehörgangsvolumen und die akustische Impedanz unter verschiedenen Bedingungen im Rahmen der Insitu-Audiometrie zu messen und dadurch individuelle Korrekturen für das Audiogramm zu berechnen bzw. Korrekturen an der Einstellung des Hörgeräts automatisch vornehmen zu können.

Die akustische Impedanz ist ein Maß für den Wirkwiderstand eines akustischen Systems. Bei niedrigen Frequenzen entspricht die akustische Impedanz in erster Näherung dem Steifigkeitsanteil der so genannten Reaktanz, der wiederum im Wesentlichen durch das abgeschlossene Luftvolumen in dem System bestimmt wird. Messtechnisch spricht man häufig von dem Kehrwert der Reaktanz, der so genannten Komplianz, deren Größe sich anschaulich durch ein Volumenäquivalent ausdrücken lässt.

Im Fall des Gehörgangs gilt es, zwei Fälle zu unterscheiden:
a) Im Fall eines schallharten Trommelfells dringt kein Schall ins Innere des Ohrs ein, d. h. die Komplianz resultiert fast ausschließlich aus dem im Gehörgang eingeschlossenen Luftvolumen. Wird über das Hörgerät ein Test-Ton ausreichend niedriger Frequenz im Gehörgang auf einen konstanten Pegel eingeregelt, lässt sich aus der dafür benötigten Ausgangsspannung am Hörgeräte-Verstärker unmittelbar das eingeschlossene Luftvolumen ablesen (siehe G. Böhme, K. Weizl-Müller: "Audiometrie", Verlag Hans Huber, 1998).
b) Im Normalfall dringt ein Teil des Schalls in das Ohr ein. Die gemessene Komplianz ist in diesem Fall größer und entspricht damit dem Äquivalent eines größeren Luftvolumens. Durch Vergleich mit der Messung im Fall eines schallharten Trommelfells lässt sich der Teil der Komplianz, der aus der Schallleitung ins Ohr resultiert, direkt berechnen.

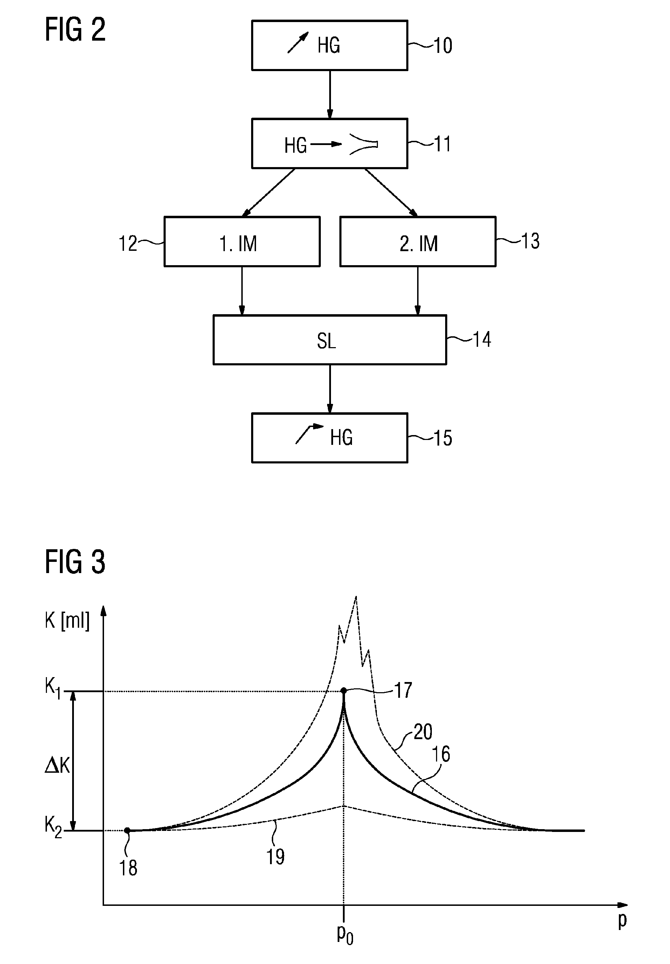

Nachfolgend wird ein beispielhafter Ablauf eines erfindungsgemäßen Anpassverfahrens anhand von FIG 2 erläutert. Zunächst wird ein Hörgerät beispielsweise mit Hilfe eines individuellen Audiogramms voreingestellt. Damit erhält das Hörgerät eine Grundeinstellung, mit der ein Hörgeräteträger wesentliche Teile seiner Schwerhörigkeit ausgleichen kann. Es ist also eine Grundeinstellung 10 gemäß FIG 2 vorzugsweise im nicht getragenen Zustand des Hörgeräts vorzunehmen. Die nachfolgende Verfeinerung der Einstellung erfolgt Insitu. Dazu wird das Hörgerät gemäß Schritt 11 in den Gehörgang eingesetzt. Dabei kann es sich beispielsweise um ein IdO-Hörgerät handeln, das komplett oder nahezu komplett in den Gehörgang eingesetzt wird. Es kann sich aber auch um ein HdO-Hörgerät handeln, bei dem nur beispielsweise ein Ohrpassstück einen Schallschlauch im Gehörgang hält. Wie unten weiter erläutert wird, muss das Ohrpassstück dann auch ein Mikrofon oder einen Schallausgangsschlauch halten.

Sobald das Hörgerät ganz oder teilweise in den Gehörgang eingesetzt ist werden zwei Insitu-Messungen 12 und 13 durchgeführt. Mit Ihnen wird die Impedanz beziehungsweise Komplianz des akustischen Systems nach dem Hörgerät, d. h. des Gehörs beziehungsweise Hörsystems festgestellt. Die erste Insitu-Messung 12 erfolgt im natürlichen Zustand des Trommelfells. Bei der zweiten Insitu-Messung 13 wird, wie unten näher erläutert werden wird, das Trommelfell schallhart gemacht, so dass im Wesentlichen Aussagen über den Gehörgangsbereich vor dem Trommelfell gemacht werden können. Aus den Messergebnissen der beiden Insitu-Messungen 12 und 13 wird eine Schallleitung 14 vom Hörgerät ins Ohr ermittelt. Mit Hilfe dieser Schallleitung 14 erfolgt schließlich eine Korrektur 15 der Einstellung des Hörgeräts.

Wie oben bereits erwähnt wurde, ist eine Voraussetzung für die Durchführung der Insitu-Messungen, dass ein zusätzliches Mikrofon ("Insitu-Mikrofon") so platziert ist, dass Schall aus dem Gehörgang aufgenommen werden kann. Beispielsweise kann ein derartiges Mikrofon zusammen mit einem Receiver räumlich eng im Hörgerät integriert sein. Es kann sich bei dem zusätzlichen Mikrofon auch um ein eigenständiges Mikrofon handeln. Dabei muss sich das Insitu-Mikrofon nicht zwingend im Gehörgang befinden, sondern kann die Signale auch durch einen geeigneten Schallschlauch zugeführt bekommen. In diesem Fall sind entsprechende Korrekturen bei der Komplianzberechnung zu berücksichtigen.

Nachfolgend sind die Verfahrensschritte ab den Insitu-Messungen 12, 13 bis zur Korrektur 15 der Hörgeräteeinstellung näher erläutert. Es ergeben sich dabei im Wesentlichen 5 Verfahrensschritte:
1. Über das Hörgerät wird ein Test-Ton geeigneter Frequenz abgegeben. Diese Frequenz sollte ausreichend niedrig sein, um zu gewährleisten, dass die gemessene Impedanz im Wesentlichen durch den Steifigkeitsterm der Reaktanz bestimmt wird. Andererseits sollte die Frequenz nicht zu niedrig sein, um zu vermeiden, dass tieffrequenter Störschall die Messung negativ beeinflusst. Darüber hinaus sollte die gewählte Frequenz kein ganzzahliges Vielfaches der üblichen Netzfrequenzen sein, um das Auftreten elektrisch induzierter Artefakte zu vermeiden. Handelsübliche Inpedanzaudiometer verwenden aus diesen Gründen eine Frequenz von 226 Hz. Der Test-Ton wird auf einen konstanten Pegel eingestellt und ermöglicht die Messung des Komplianzanteils des funktionalen Ohrs. Für die Messung sind ggf. vorhandene Ausgleichsbohrungen (Vent) des Hörgeräts temporär zu verschließen. Über ein Insitu-Mikrofon wird der im Gehörgang anliegende Schallpegel registriert. Diese erste Insitu-Messung 12 wird unter natürlichen Bedingungen für das Trommelfell durchgeführt.
2. Der Hörgeräteträger wird für die zweite Insitu-Messung 13 gebeten, durch ein so genanntes Valsava-Manöver, also durch eine Lufteinpressung über die Eustachische Röhre, den Luftdruck im Mittelohr zu erhöhen, wodurch das Trommelfell schallhart vorgespannt wird. Analog zu dem obigen Schritt 1. wird so über das Insitu-Mikrofon die Komplianz des Gehörgangs gemessen, woraus sich das eingeschlossene Luftvolumen unmittelbar berechnen lässt.
3. Durch das in Schritt 2. ermittelte Volumen lässt sich abschätzen, ob der Gehörgang für die Messung ausreichend abgeschlossen ist, oder ob durch schlechten Sitz des Geräts oder durch eventuell vorhandene Ausgleichsbohrungen die Messung verfälscht wurde. Die Abschätzung erfolgt durch Vergleich gegenüber bekannten Werten des Gehörgangsvolumens.
4. Durch das in Schritt 2. ermittelte Luftvolumen lässt sich durch Kenntnis des Gehörgangsdurchmessers die Länge des Gehörgangs abschätzen. Aus dieser Längenschätzung lassen sich bei der Insitu-Audiometrie über frequenzabhängige Korrekturen die resultierenden Pegel in der Ebene des Trommelfells berechnen. Die resultierenden Pegel ergeben sich für einen individuellen Gehörgang beispielsweise durch Auslöschungen, stehende Wellen und dergleichen. Der Gehörgangsdurchmesser für die Schätzung der Länge des Gehörgangs ist durch die Bauform des Hörgeräts oder des Ohrpassstücks bekannt.
5. Durch die Differenz der in den Schritten eins und zwei ermittelten Komplianzen lässt sich der Anteil des Schalls berechnen, der in das Mittelohr eindringt (Mittelohrkomplianz; Schallleitung 14, vergleiche FIG 2). Dabei können zwei Fälle auftreten:
   5a) Die Mittelohrkomplianz ist sehr niedrig. Dies deutet auf eine Versteifung der ossikularen Kette oder des Trommelfells hin. Über dieses Ergebnis lässt sich abschätzen, ob und in welchem Maß die Luftleitung des Hörgeräteträgers gestört ist. Diese Kenntnis kann genutzt werden, um eine Schallleitungs-Komponente bei der Insitu-Audiometrie abschätzen zu können. Im Falle einer Störung der Luftleitung muss beispielsweise eine breitbandige zusätzliche Verstärkung erfolgen.
   5b) Die Mittelohrkomplianz ist deutlich erhöht. In diesem Fall liegt wahrscheinlich eine Unterbrechung der ossikularen Kette vor. Analog zum Fall 5 a) ist auch in diesem Fall die Schallleitung gestört und es ist möglich, das Insitu-Audiogramm durch einen breitbandigen Korrekturfaktor zu ergänzen.

In FIG 3 ist ein Tympanogramm dargestellt, in dem die durchgeführten Insitu-Messungen in ihren Messergebnissen wiedergegeben sind. Die durchgezogene Linie 16 stellt ein typisches Tympanogramm dar. Bei einem Normaldruck P₀ stellt sich ein Resonanzwert K₁ ein. Dieser Wert K₁ kann durch die erste Insitu-Messung 12 bei natürlicher Spannung des Trommelfells gemessen werden (Messpunkt 17). Ein weiterer Messpunkt 18 wird durch die zweite Insitu-Messung 13 ermittelt, bei der das Trommelfell durch Lufteinpressung vorgespannt ist. Es resultiert daraus ein Komplianzwert K₂ der beispielsweise um den Faktor fünf bis zehn kleiner ist als der Komplianzwert K₁. Aus der Differenz der beiden Komplianzwerte K₁ und K₂ ergibt sich die durch das Luftvolumen zwischen Hörgerät und Trommelfell bestimmte Komplianz ΔK. Sie stellt den Wert für dieses Luftvolumen dar. Für diesen Wert ΔK gibt es gewisse Erfahrungswerte. Weicht ein gemessenes ΔK deutlich von diesem Erfahrungswert ab, so kann von einer Beschädigung des Mittelohrs bzw. Trommelfells ausgegangen werden. Ist beispielsweise das Trommelfell gerissen, so liegt der zweite Messpunkt 18 deutlich höher und der Wert für ΔK ist entsprechend gering. Für ältere Probanten ergibt sich häufig ein Tympanogramm gemäß der Kurve 19. Die Schallleitung ist dabei durch weniger mobile Knöchelchen im Mittelohr behindert. Es kann aber auch eine Cerumen-Verstopfung des Gehörgangs vorliegen. Der erste Messpunkt 17 würde dann deutlich niedriger liegen, wodurch sich ebenfalls ein sehr kleiner Wert ΔK ergibt.

Liegt eine Unterbrechung der Knöchelchenkette im Mittelohr vor, ergibt sich unter Umständen ein Tympanogramm gemäß Kurve 20. Dann liegt der Messpunkt 17 üblicherweise deutlich höher, und der Komplianzwert ΔK liegt wesentlich über dem Erfahrungswert. Anhand der ermittelten Werte ΔK kann dann automatisch eine Korrektur der Signalverarbeitung des Hörgeräts durchgeführt werden. Beispielsweise kann eine zusätzliche breitbandige Verstärkung oder auch nur eine zusätzliche Verstärkung in gewissen Frequenzbereichen appliziert werden. Ebenso kann die Verstärkung in gewissen Frequenzbereichen reduziert werden, in denen aufgrund einer Mittelohrerschädigung keine Frequenzen übertragen werden können. Dadurch lässt sich die Lebensdauer der Hörgerätebatterie verlängern.

Das erfindungsgemäße Verfahren zur Anpassung eines Hörgeräts wird vorzugsweise im Vorfeld einer Insitu-Audiometrie eingesetzt und weist mehrere Vorteile auf. Es lässt sich nämliche eine individuelle Gehörgangskorrektur berechnen, die die Qualität des Insitu-Audiogramms verbessert. Zusätzlich lässt sich diese Korrektur bei der Berechnung der Zielverstärkung des Geräts nutzen und ermöglicht so eine optimale Anpassung des Hörgeräts. Darüber hinaus ermöglicht das Verfahren eine Abschätzung der Schallleitung-Komponente des Insitu-Audiogramms und damit eine verbesserte Berechnung der Zielverstärkung des Hörgeräts. Ferner lässt sich mit Hilfe des erfindungsgemäßen Verfahrens der korrekte Sitz des Hörgeräts überprüfen. Diese Überprüfung lässt sich auch in Alltagssituationen, z. B. nach dem Einsetzen des Geräts, wiederholen.

## Patentansprüche

1. Verfahren zum Anpassen eines Hörgeräts an einen Nutzer durch
- Einstellen (10) des Hörgeräts individuell für den Nutzer,
und
- Einsetzen (11) des Hörgeräts zumindest teilweise in den Gehörgang des Nutzers,
- Insitu-Messen (12, 13) der akustischen Impedanz des Hörsystems des Nutzers einschließlich zumindest eines Teils des Gehöhrgangs des Nutzers und
- automatisches Korrigieren (15) der individuellen Einstellung des Hörgeräts anhand des Ergebnisses der Insitu-Messung,
**dadurch gekennzeichnet, dass**
- das Insitu-Messen mit einer tympanometrischen Methode erfolgt,
- das Trommelfell für die Insitu-Messung vorübergehend schallhart gemacht wird und
- eine Differenz von zwei Insitu-Messungen (12, 13), eine bei schallhartem Trommelfell und die andere bei nicht schallhartem Trommelfell, zu dem automatischen Korrigieren (15) der Einstellung verwendet wird.

2. Verfahren nach Anspruch 1, wobei die individuelle Einstellung (10) des Hörgeräts mit einem Audiogramm erfolgt.

3. Verfahren nach Anspruch 1 oder 2, wobei das Hörgerät einen Vent aufweist, der für die Insitu-Messung (12, 13) temporär verschlossen wird.

4. Verfahren nach einem der vorhergehenden Ansprüche, wobei bei der Insitu-Messung (12, 13) ein Test-Ton konstanten Pegels mit einer Frequenz zwischen 200 Hz und 300 Hz von dem Hörgerät abgegeben wird.

5. Verfahren nach einem der vorhergehenden Ansprüche, wobei aus der Insitu-Messung (12, 13) ermittelt wird, ob der Gehörgang in einem vorgegebenen Maß verschlossen ist.

6. Verfahren nach einem der vorhergehenden Ansprüche, wobei aus der Insitu-Messung (12, 13) das Luftvolumen und daraus die Distanz zwischen Hörgerät und Trommelfell geschätzt wird, und anhand der Distanz ein Schallpegel eines vom Hörgerät abgegebenen Test-Tons am Trommelfell für das Korrigieren der individuellen Einstellung des Hörgeräts ermittelt wird.

7. Hörgerät mit
- einer Signalverarbeitungseinrichtung, die individuell für einen Nutzer einstellbar (10) ist, und
- einem Abschnitt, der in einem Gehörgang des Nutzers einsetzbar (11) ist,
- einer Messeinrichtung, mit der die akustische Impedanz des Hörsystems des Nutzers einschließlich zumindest eines Teils des Gehörgangs des Nutzers im in den Gehörgang eingesetzten Zustand des Abschnitts insitu messbar (12, 13) ist, wobei
- die individuelle Einstellung der Signalverarbeitungseinrichtung anhand der tympanometrischen Messung automatisch korrigierbar (15) ist,
**dadurch gekennzeichnet, dass**
- die Messeinrichtung dazu ausgebildet ist, die akustische Impedanz mit einer tympanometrischen Methode zu messen, und
- eine Differenz von zwei Insitu-Messungen (12, 13), eine bei schallhartem Trommelfell und die andere bei nicht schallhartem Trommelfell, zu dem automatischen Korrigieren (15) der Einstellung verwendet wird.

8. Hörgerät nach Anspruch 7, wobei die Messeinrichtung ein Mikrofon umfasst, das in den in den Gehörgang einsetzbaren Abschnitt des Hörgeräts integriert ist.
